# EUROPÄISCHE PATENTSCHRIFT

(11) **EP 1 362 611 B1**
(45) Veröffentlichungstag und Bekanntmachung des Hinweises auf die Patenterteilung: **08.12.2004**
(21) Anmeldenummer: 02011080.5
(22) Anmeldetag: 17.05.2002
(51) Int. Cl.: A61M 16/10, A61M 15/02

(54) **Therapiegerät zur Behandlung von Erkältungen**
Therapy device for treatment of colds
Appareil thérapeutique pour les soins des rhums

(43) Veröffentlichungstag der Anmeldung: 19.11.2003
(73) Patentinhaber: Vitaya Patent GmbH, 14050 Berlin (DE)
(72) Erfinder: Opitz, Christian, Tucson, AZ, 85750 (US)
(74) Vertreter: Müller - Hoffmann & Partner

(56) Entgegenhaltungen:
- DE-B- 1 036 470
- US-A- 3 362 405
- US-A- 4 699 136
- DATABASE WPI Derwent Publications Ltd., London, GB; AN 2000-658137 XP002216438 & HU 9 802 379 A (KALMAN D ET AL) 28. September 2000 (2000-09-28)
- DATABASE WPI Derwent Publications Ltd., London, GB; AN 1995-274266 XP002216439 -& SU 1 748 328 A (LUFEROV A V) 20. Februar 1995 (1995-02-20)
- DATABASE WPI Derwent Publications Ltd., London, GB; AN 1994-301189 XP002216440 -& SU 1 814 905 A (BASHKIRSKIJ SELSKOKHOZ I) 15. Mai 1993 (1993-05-15)
- DATABASE WPI Derwent Publications Ltd., London, GB; AN 2001-030536 XP002216441 -& RU 2 153 895 C (VISHNEVSKAJA NINA LEONIDOVNA) 10. August 2000 (2000-08-10)

## Beschreibung

Die Erfindung betrifft ein Gerät zur Therapierung und Heilung von Erkältungssymptomen und -krankheiten bei Menschen, insbesondere im Nasen-, Stirnhöhlen- und Rachenbereich.

Als Ergebnis umfangreicher virologischer Forschung ist es bereits seit etwa 1971 bekannt, das sich das Rhinovirus, welches bereits damals als Ursache für gewöhnliche Erkältungen bekannt war, bei normaler Körpertemperatur von 37 °C nicht vermehren kann; vergleiche Lit. [3] der beigefügten Literaturliste. Obgleich alle infektiöse Krankheiten verursachenden Viren zellspezifisch sind, entpuppte sich das Rhinovirus als einzigartig insofern, als es nur innerhalb der Epithelschichten der Nasen- und Rachenwege existieren kann, d. h. in Körperregionen, die durch die einströmende Luft beim Einatmen eine geringere Temperatur aufweisen als das Körperinnere, insbesondere als die menschliche Körperkerntemperatur. Die Möglichkeit, gewöhnliche Erkältungen zu heilen, wurde damals übersehen, jedenfalls hinsichtlich eines Therapieansatzes nicht weiter untersucht, da nicht bekannt war, welcher Prozentsatz aller Erkältungen durch das Rhinovirus bzw. das Coronavirus verursacht werden. Bei Letzterem wurde ebenfalls festgestellt, dass es ähnlich hitzesensibel ist wie das Rhinovirus.

In einer im Jahr 1980 am Harbord-Krankenhaus im englischen Salisbury durchgeführten Studie stellten Larson, Reed und Thyrrell fest, dass etwa 90 % aller Erkältungen durch diese beiden genannten Viren verursacht werden, wobei das Rhinovirus zu etwa 70 % und das Coronavirus zu etwa 20 % beteiligt sind; vergleiche Lit. [4]. Ferner ist bekannt, dass das Coronavirus nur zwei Subtypen ausbildet, während mittlerweile 89 Subtypen des Rhinovirus klassifiziert sind und von mehreren hundert weiteren noch zu erforschenden Subtypen ausgegangen wird. Wichtig ist, dass alle Subtypen der Rhino- und Coronaviren bei Temperaturen über 37 °C nicht überleben können. Der menschliche Körper selbst nutzt den Vorteil der Wärmeempfindlichkeit der Rhino- und Coronaviren bei seiner Abwehr gegen Erkältungen. Die typische Epithelschwellung der Nasenhöhlen, verursacht durch eine erhöhte Histaminproduktion, erlaubt es einem unter Erkältung Leidenden gerade deshalb nicht durch die Nase zu atmen, weil der Körper verhindern will, dass die einströmende Luft das Epithel abkühlt. Dadurch wird die Vermehrung der Viren verlangsamt und innerhalb von 3 bis 4 Tagen erreicht die Interferon-Produktion des Körpers einen Stand, der es ermöglicht, die Eindringlinge zu besiegen. Normalerweise erfolgt die Temperaturregulation der Nasengänge und Nasennebenhöhlen durch den Einstrom kühler Luft und die Abgabe von Wärme über das Gehirn. Das Verschießen der Nasenwege gegenüber einströmender Luft führt zu der bekannten unangenehmen Erwärmung des Gehirns. Dies erklärt die von vielen Menschen während einer Erkältung empfundene Schläfrigkeit.

Die gesteigerte Schleimproduktion ist ein weiterer Abwehrmechanismus des Körpers in dem Versuch, sich des Virus' zu entledigen. Dieser Prozess dehydriert den Körper, indem eine ungewöhnlich hohe Menge an Wasser den Nasengängen zugeleitet wird. Als Folge der Dehydration kommt es zu einer Ansammlung von Milchsäure und anderen "Abfallprodukten". Hieraus können Schmerzen im gesamten Körper resultieren. In Anbetracht dieser Reaktionen des Körpers auf Erkältungen wird aber auch deutlich, warum übliche Nasensprays und andere Mittel, welche die Nasengänge vorübergehend öffnen, eher kontraproduktiv sind und eine Erkältung verlängern. Das Austrocknen des Schleims, das Reduzieren der Schwellung und das erneute Einströmen kalter Luft stört die Abwehrmechanismen des Körpers und erhöht so die Überlebenschancen der Viren.

Die erwähnte Entdeckung der Hitzeempfindlichkeit von Rhino- und Coronaviren führte zu verschiedenen Wärmeanwendungen bei der Behandlung von Erkältungen. Dabei wurde die Erkenntnis ausgenutzt, dass die genannten Viren bei Lufttemperaturen von 50 bis 53 °C binnen Minuten absterben. Zur Behandlung von Erkältungen wurden daher sogenannte Gesichtssaunageräte für die isolierte Behandlung des Kopfes entwickelt. Auch werden seit langem zumindest in Europa trockene Saunen von sogenannten ganzheitlichen Therapeuten empfohlen.

Aus US 4 699 136 bzw. US 5 038 769 ist ein nach Art eines Handföhns gestaltetes Gerät zur lokalen Hyperthermie-Behandlung von Erkältungskrankheiten, Asthma und dgl. bekannt, wobei der den Atmungsbereich des Gesichts beaufschlagende Warmluftstrom von beispielsweise 41 bis 44 °C zusätzlich mit einem durch eine Zerstäubervorrichtung zugemischten Medikamentenanteil beladen wird.

Während die bekannten Therapien bei wiederholter Anwendung die Symptome von Erkältungen zum Verschwinden bringen können, haben sie dennoch einige gravierende Nachteile. Die Behandlung in einer trocknen Sauna kann normalerweise nur einmal am Tag ausgehalten werden. Es ist jedoch unmöglich, alle Viren bei nur einer Anwendung von heißer Luft pro Tag zu töten, da inkubierte und kristallisierte Viren dabei zunächst nicht zerstört werden. Eine Dampfinhalation ist zusätzlich mit dem Nachteil verbunden, dass sie das Epithel äußerlich befeuchtet und damit den Körper dazu anregt, die eigene, der Abwehr dienende Schleimproduktion zu reduzieren. Lokale Warmluft-Hyperthermie mit Medikamenten-Aerosolzusatz gemäß den genannten US-Patentschriften ist wegen vergleichsweise hohen Medikamentenverbrauchs kostenintensiv und fast nie frei von Nebenwirkungen. Trockene Saunen und Dampfinhalationen können zwar etwas Erleichterung verschaffen, aber keine Heilung bewirken. Gesichtssaunen mit oder ohne Dampfinhalationen sowie die Warmluft-Hyperthermie mit eingesprühten Medikamenten haben den Nachteil, dass sie die Verteilung von Chi im Körper, also das Gleichgewicht von biochemischen und bioelektrischen Korrelationen aus dem Gleichgewicht von biochemischen und bloelektrischen Korrelationen aus dem Gleichgewicht bringen, indem sie den ganzen Kopf überwärmen und damit aus der Sicht der traditionellen chinesischen ganzheitlichen Medizin das Yin, d. h. den Flüssigkeitshaushalt des Körpers, stören und die Wirksamkeit blutspezifischer Heilkräfte schwächen.

Ein anderer, älterer Therapieansatz, dem das in US 3,363,405 beschriebene Gerät zugrunde liegt, nutzt die Beobachtung aus, dass Menschen, die unter Stim- und -nebenhöhlenerkrankungen leiden, im Allgemeinen bei längerem Aufenthalt in einem Seeklima mit gewissem, geringen Salzgehalt in der Luft Besserung empfinden. Demgemäß schlägt diese US-PS vor, ein solches Seeklima in einem umgrenzten Behandlungsraum dadurch nachzubilden, dass ein durch einen Lüfter umgewälzter Luftstrom über einen am Boden einer Kammer im Gerät enthaltene Charge, vorzugsweise aus Meersalz, geführt wird und dadurch um einen in etwa dem Seeklima entsprechenden ganz geringen Salzgehalt angereichert wird. Zur Behandlung von akuten, durch das Rhino- bzw. Koronavirus verursachten Erkältungen ist dieser Therapieansatz praktisch wirkungslos.

Auf diesem kurz geschilderten Wissenshintergrund liegt der Erfindung die Aufgabe zugrunde, ein preiswertes leicht zu handhabendes Gerät zur therapeutischen Behandlung und Heilung von Erkältungen zu schaffen.

Die Erfindung bezieht sich auf ein Therapiegerät gemäß Auspruch 1.

Die Warmluftstrom-Erzeugungseinrichtung ist vorzugsweise als Handgerät nach Art eines Haarföhns gestaltet und der vom generierten Warmluftstrom beaufschlagte Körper ist als eine in diesem Luftstrom angeordnete Matrix aus dem kristallisierten Salz gestaltet.

Der vom Warmluftstrom beaufschlagte Körper aus kristallisiertem Salz oder dieses Salz enthaltend kann auch in einem auf das Handgerät, also insbesondere einen Haarföhn herkömmlicher Bauart ansetzbaren oder aufsteckbaren Adapter vorzugsweise auswechselbar eingebaut sein.

Weiterhin kann der das kristallisierte Salz enthaltende oder aus diesem Salz geformte Körper beispielsweise und insbesondere als auswechselbares scheibenförmiges Gitter gestaltet und quer zur Warmluftströmung und in mehr oder weniger loser Schüttung gehalten sein. Für die Formgestaltung des Körpers aus kristallinem Salz bestehen die unterschiedlichsten Möglichkeiten innerhalb der Grenzen der geometrischen Vorgabe durch das Warmluft-Erzeugungsgerät, also insbesondere des Föhns oder den zugeordneten aufsteckbaren Adapter. Um eine möglichst effektive Freisetzung von erwünschten negativ ionisierten Luftionen im Warmluftstrom zu erreichen, kann der Körper aus kristallisiertem Salz - wie erwähnt - eine quer zur Luftströmung auswechselbar angeordnete, beispielsweise durch eine gitterartige Umhüllung gehaltene luftdurchlässige kristallisierte Salzmischung sein. Noch laufende Versuche haben aber auch gezeigt, dass eine hülsenoder ringförmige Gestaltung des Körpers aus kristallisiertem Salz zur erwünschten Vorbehandlung des erwärmten Luftstroms ausreichen kann, wobei der kristallisierte Salzkörper entweder gepresst oder vorzugsweise mit einem Kunststoffanteil verpresst als Formkörper hergestellt werden kann. Gute Ergebnisse hinsichtlich der Ionisierung des warmen Luftstroms werden auch erzielt, wenn der Körper aus kristallisiertem Salz, in Luftströmungsrichtung gesehen, als Rundhülse mit sich verjüngendem Querschnitt gestaltet ist bzw. insbesondere die Form einer Laval-Düse erhält, wodurch zusätzlich eine Konzentration und Erhöhung der Austrittsgeschwindigkeit des vom kristallisierten Salz ionisierten Warmluftstroms erreicht wird. Durchgeführte Untersuchungen und Tests haben ergeben, dass grundsätzlich eine minimale Berührung des Warmluftstroms mit dem kristallisierten Salz ausreicht, um den gewünschten Effekt einer negativen Ionisierung bei gleichzeitiger Verstärkung des Skalarwellenfelds zu erreichen. Als zweckmäßig hat sich dabei eine ringförmige Umhüllung, insbesondere und beispielsweise hergestellt als Spritzgussformteil aus temperaturbeständigem Kunststoff erwiesen, die das Salz als Granulat enthält und als Kranz an oder im Bereich der Öffnung des Geräts bzw. des Adapters angebracht wird.

Geht man von der obigen Feststellung aus, dass sich Erkältungsviren prinzipiell durch einen erwärmten Luftstrom abtöten lassen, so könnte die Verwendung eines normalen Haarföhns zwar effektiv sein um Erkältungssymptome zu beseitigen. Langfristig jedoch ist dessen Anwendung der Gesundheit, aber auch einer vollständigen Wiedergenesung abträglich. Hierbei spielt eine Rolle, dass Föhne üblicherweise elektromagnetische Felder in einer Größenordnung von 700 mG erzeugen. Dieser Wert liegt weit über dem von vielen Fachleuten als sicher angesehenen Wert von 30 mG. Hierbei ist auch zu berücksichtigen, dass das elektromagnetische Eigenfeld einer Person während einer Erkältung bereits geschwächt ist, da der Körper Energie für den Heilungsprozess bereitstellt - ein Vorgang, der insgesamt wesentlich komplexer ist als die Eliminierung des Erkältungsvirus. Die Regeneration des Epithels nach einer Erkältung dauert normalerweise zwischen 40 und 60 Tagen. Dieser Prozess wird durch eine Störung des elektromagnetischen Eigenfelds, verursacht durch die elektromagnetischen Felder des Haarföhns, empfindlich gestört. Kinder sind gegenüber hohen elektromagnetischen Feldern besonders anfällig und erkranken nach allgemeiner Erfahrung etwa zweimal so häufig an Erkältungen wie Erwachsene. Eine weitere wesentliche Rolle spielt auch, dass normale Haarföhne positive Luftionen (freie Radikale) in der von ihnen ausgestoßenen Luft produzieren. Diese werden ebenfalls als gesundheitsschädigend angesehen, insbesondere wenn die aus dem Föhn austretende Luft direkt eingeatmet wird, was jedoch zur Bekämpfung der Erkältung notwendig wäre. Positive Ionen in der Luft machen den Körper zudem anfällig gegenüber bakteriellen Infektionen und begrenzen so den Wert einer Behandlung mit der von normalen Haarföhnen produzierten Heißluft.

Die Erfindung geht über diese reine Warmlufttherapie durch mindestens eine oder mehrere gezielte technische Zusatzmaßnahmen hinaus:
Zum Einen wird die einen Warmluftstrom generierende Einrichtung, also insbesondere der schon erwähnte Haarföhn, in Verbindung mit einer relativ kleinen Menge an kristallisiertem Salz verwendet, das mit dem Warmluftgerät in einer solchen Weise verbunden wird, dass sich die ausgestoßene Warmluft über das Salz bewegt und mit diesem in einen mehr oder weniger innigen Austausch gerät.

Hinsichtlich weiterer Einzelheiten dieser Zusatzmaßnahme wird auf die obige Darstellung, die beigefügten Patentansprüche sowie auf die nachfolgende Beschreibung von Ausführungsbeispielen verwiesen.

Als vorteilhafte Zusatzmaßnahme wird das Prinzip eines Haarföhns mit einer ungewöhnlich geringen Produktion an elektromagnetischen Feldern verwendet. Haartrocknungsgeräte mit einer sehr geringen externen Abstrahlung von elektromagnetischen Feldern im Bereich von beispielsweise nur 2 mG gibt es als solche bereits. Diese werden beispielsweise hergestellt und vertrieben von der Firma EMHHC (Electromagnetic Health Hazard Control) in Pacific Palisades, Kalifornien/USA.

Ein gemäß der Lehre der Erfindung für die Behandlung von Erkältungen eingerichteter Föhn, insbesondere mit einem erfindungsgemäßen Adapter zu versehender, könnte natürlich auch in seiner üblichen Funktion, nämlich zur Trocknung von Haaren eingesetzt werden, ohne dass sich der Nutzer dabei den schädlichen Wirkungen hoher EMFs aussetzt.

Das gemäß der Erfindung zum Einsatz kommende kristallisierte Salz aus natürlichen Vorkommen ist schon seit einigen Jahrhunderten mindestens in Europa als das "königliche Salz" bekannt, da seine Verwendung ausschließlich fürstlichen Häusern vorbehalten blieb. In der Natur kommt es nur zu etwa 1 % des Salzvorkommens in Salzminen, beispielsweise in Bad Reichenhall (DE) oder in den Salzminen bei Krakau (PL), auch bekannt als "Königs-Steinsalz", vor. In vielen Salzminen überhaupt nicht. Die chemische Zusammensetzung und die Eigenschaften des kristallisierten Salzes sind überaus erstaunlich und übertreffen bei weitem diejenigen von gewöhnlichem Meer- oder Steinsalz. Kristallisiertes Salz, wie es im Zusammenhang mit der Erfindung vorzugsweise zu verwenden ist, enthält in der Regel alle 81 Elemente, aus denen der menschliche Körper besteht. Es produziert ein permanent ausgeglichenes Energiefeld (Meißnerfeld), dass es insbesondere diskohärente, beispielsweise durch AC-betriebene Geräte verursachte elektromagnetische Felder zu Kohärenz bringt. Nicht zuletzt aus diesem Grund werden nukleare Abfälle in alten, nicht mehr genutzten Salzminen gelagert, die kristallisiertes Salz enthalten. Die normalen Eigenschaften von natürlich vorkommendem Salz treffen nur teilweise in Bezug auf die kristallisierte Form zu. So wird durch und über die kristallisierte Salzart eine große Menge an Skalarwellen generiert - jene Wellenform mittels der alle Zellen des menschlichen Körpers kommunizieren. Dadurch gelangen ausgleichende Wirkungen des kristallisierten Salzes bis tief in den menschlichen Körper.

Es wurde beobachtet, dass die ionisierende Wirkung des Salzes und hier insbesondere des kristallisierten Salzes den Körper bei der Regeneration des Epithels unterstützt, wodurch die Wirkung der Behandlung auch auf sekundäre bakterielle Infektionen ausgedehnt wird. Dadurch lassen sich nicht nur die akuten durch eine virale Infektion verursachten Symptome behandeln, sondern zusätzlich wird die Gesundung und Gesundheit der Nasen- und Nasennebenhöhlen im Allgemeinen gefördert. Damit wird zusätzlich eine bessere Widerstandsfähigkeit gegenüber Erkältungen und anderen Infektionen bewirkt und damit die Wahrscheinlichkeit zukünftiger Erkrankungen verringert.

Die Erfindung und vorteilhafte Einzelheiten werden nachfolgend unter Bezug auf die Zeichnungen in beispielsweisen Ausführungsformen näher erläutert. Es zeigen:
- **Fig. 1**: eine Prinzip-Schnittbilddarstellung eines Therapiegeräts zur Behandlung von Erkältungen mit erfindungsgemäßen Merkmalen;
- **Fig. 2 bis 6**: vorteilhafte Ausgestaltungen des erfindungsgemäß vorgesehenen durch den Warmluftstrom vor Austritt aus dem Gerät beaufschlagten, als austauschbarer Einsatz gestalteten Körpers der kristallisiertes Salz enthält oder aus diesem besteht;
- **Fig. 7**: eine vergrößerte Schnittdarstellung gesehen in Richtung der Pfeile A-A' in Fig. 4;
- **Fig. 8**: eine Vorsatzhülse mit zugeordnetem Adapter zur Anwendung der Erfindung bei Verwendung eines herkömmlichen Haarföhns; und
- **Fig. 9**: eine abgewandelte Ausführungsform einer Vorsatzhülse mit erfindungsgemäßem salzhaltigem Ionisierungselement und zugeordnetem Adapter.

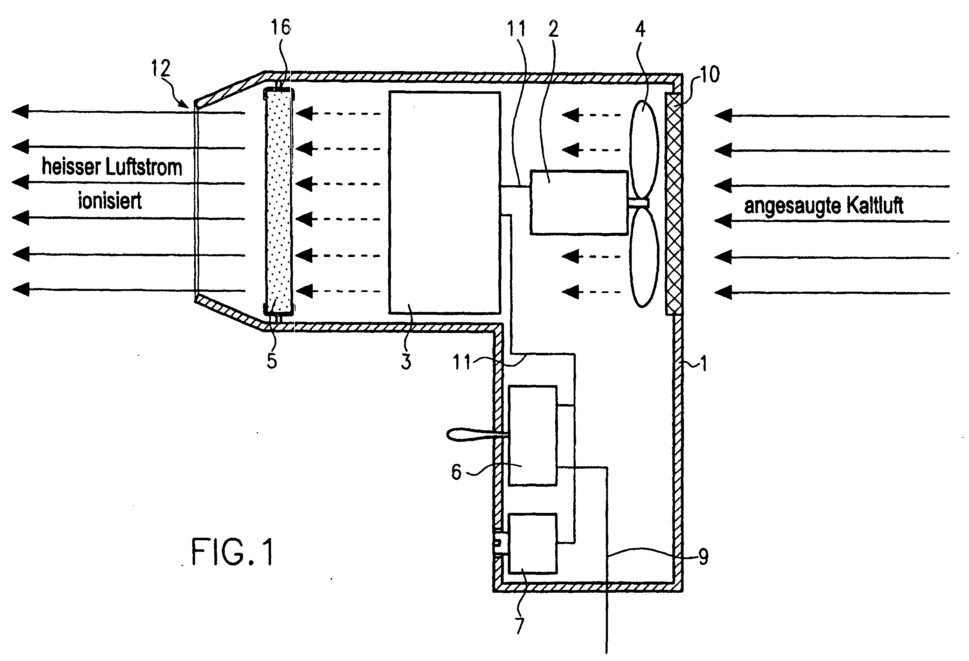

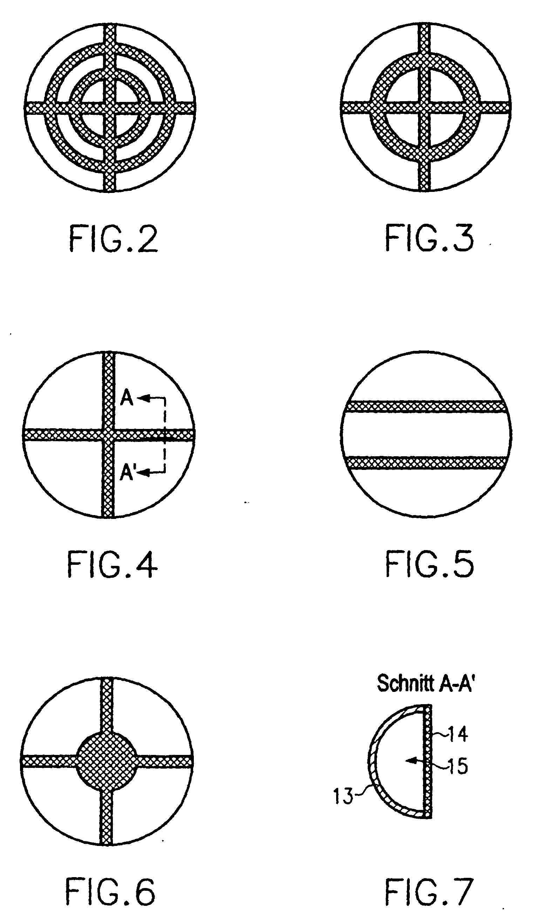

Die Fig. 1 zeigt die Prinzip-Schnittdarstellung eines zur Behandlung von Erkältungen geeigneten Therapiegeräts in der Ausgestaltungsform als Föhn. In einem in der Regel schon aus Kostengründen als Spritzteil gefertigten abgewinkelten Gehäuse 1 aus form- und temperaturstabilem Kunststoff, nämlich insbesondere dem Gehäuse eines herkömmlichen Haarföhns sind - jeweils in schematischer Andeutung - folgende Baugruppen bzw. Bauteile in der angedeuteten Gruppierung, Anordnung und gegenseitigen Zuordnung untergebracht: Ein Gebläsemotor 2, der ein Flügel- oder Schraubengebläse 4 antreibt, durch welches kalte Umgebungsluft vorzugsweise über ein Gitter oder ein Flusensieb 10 auf der (im Bild rechten) Rückseite angesogen wird. Die angesaugte Kaltluft strömt durch oder über ein bekanntes Heizelement 3 in Form einer Heizwendel oder eines Heizstabs oder -gitters und wird dann als heißer Luftstrom über eine Frontöffnung 12 auf der der Ansaugseite gegenüberliegenden Seite ausgestoßen. Erfindungsgemäß ist in Luftströmungsführung nach dem Heizelement 3 ein schematisch angedeuteter Körper 5 angeordnet, der aus kristallisiertem Salz besteht oder einen hohen Anteil an diesem Salz enthält und vom heißen Luftstrom durchsetzt oder durchströmt oder mindestens gestreift wird. Wie in der Zeichnung von Fig. 1 angedeutet, kann der Körper 5 als rundes, scheibenförmiges Matrixelement gestaltet und quer zur warmen Luftströmung angeordnet sein, wobei der Ausdruck Matrixelement bedeutet, dass entweder Salzkristalle in ein gitterartiges scheibenförmiges Element, beispielsweise aus Kunststoff, in mehr oder weniger loser Schüttung eingebracht sind, so dass beim Durchströmen der heißen Luft eine mehr oder weniger innige Kontaktberührung zwischen den Salzkristallen und der durchströmenden Luft entsteht. Eine andere Gestaltungsmöglichkeit für das Matrixelement besteht darin, Salzkristalle mit gut verformbaren Kunststoffanteilen als scheibenförmiges verpresstes Element herzustellen, das auswechselbar in den vorderen Warmluft-Austrittsbereich des Föhngehäuses eingesetzt werden kann. Eine weitere Möglichkeit besteht darin, den Körper aus kristallisiertem Salz bzw. eingelagertem kristallisiertem Salz in Form einer röhrenförmigen Hülse zu gestalten, die in den vorderen Warmluft-Austrittsbereich des Gehäuses 1 auswechselbar eingebaut ist. Dieser hülsenförmige Körper kann in Luftströmungsrichtung gesehen auf der Luft-Abströmseite verjüngt, insbesondere in Form einer Laval-Düse gestaltet sein, wodurch eine gute Luftströmungsführung und ein inniger Kontakt der durchströmenden Luft mit dem kristallisierten Salz erreicht wird, so das eine erwünschte gute negative Ionisierung des warmen Luftstroms erreicht werden kann. Weitere Ausführungsbeispiele für eine mögliche Formgestaltung des Ionisierungskörpers veranschaulichen die schematischen Zeichnungen der Figuren 2 bis 7, auf die weiter unten noch Bezug genommen wird.

Im unteren Handgriffbereich des Gehäuses 1 ist ein vorzugsweise mehrstufiger üblicher Netzschalter 6 und gegebenenfalls ein Netzspannungsumschalter 7 vorhanden. Bezugshinweis 9 bezeichnet eine Stromzuführungsleitung; die geräteinterne Verdrahtung ist durch Bezugshinweis 11 gekennzeichnet.

Es ist von besonderem Vorteil, wenn das die Warmluftströmung erzeugende Gerät, also insbesondere der Haarföhn, möglichst niedrig extern abgestrahlte elektromagnetische Felder erzeugt. Um dies zu erreichen, sind verschiedene, insbesondere passive Maßnahmen bekannt, wie die Verwendung von HF-Filtern, EM-Abschirmmaßnahmen, Verwendung symmetrischer Leitungsverdrahtung und dergleichen. Es kann auch ein sogenanntes Resource-Element in der Form eines geometrischen Struktur- und Informations-Resonanzschlüssels vorgesehen werden, durch welches die entstehenden elektromagnetischen Wellen in eine naturkohärente Form umgewandelt werden.

Ist vorgesehen - wie in Fig. 1 dargestellt, den ionisierenden Körper 5 aus kristallisiertem Salz im vorderen Austrittsbereich des heißen Luftstroms anzuordnen, so kommen dafür unterschiedliche Gestaltungsmöglichkeiten in Frage. Beispiele zeigen die Fig. 2 bis 6. Bei diesen ist der kristalline Salzkörper in ein gitter- oder käfigartiges Kunststoffteil eingebracht, dessen Längs- und Querrippen, wie die Fig. 7 veranschaulicht, als Spritzgussformteil 13 aus temperaturbeständigem Kunststoff bestehen kann, der auf der vom zuströmenden Luftstrom beaufschlagten Seite einer luftdurchlässigen Abdeckung, beispielsweise aus einem Kunststoffgewebe 14, vorgesehen ist. Im Inneren dieses durch das Kunststoffgewebe 14 abgedeckten Spritzgussformteils 13 befindet sich das Kristallsalz 15. Das gesamte Bauteil 5 in Fig. 1 ist auswechselbar im vorderen Luftaustrittsteil des Warmlufterzeugungsgeräts gehalten, beispielsweise mittels einer Klemmhalterung 16.

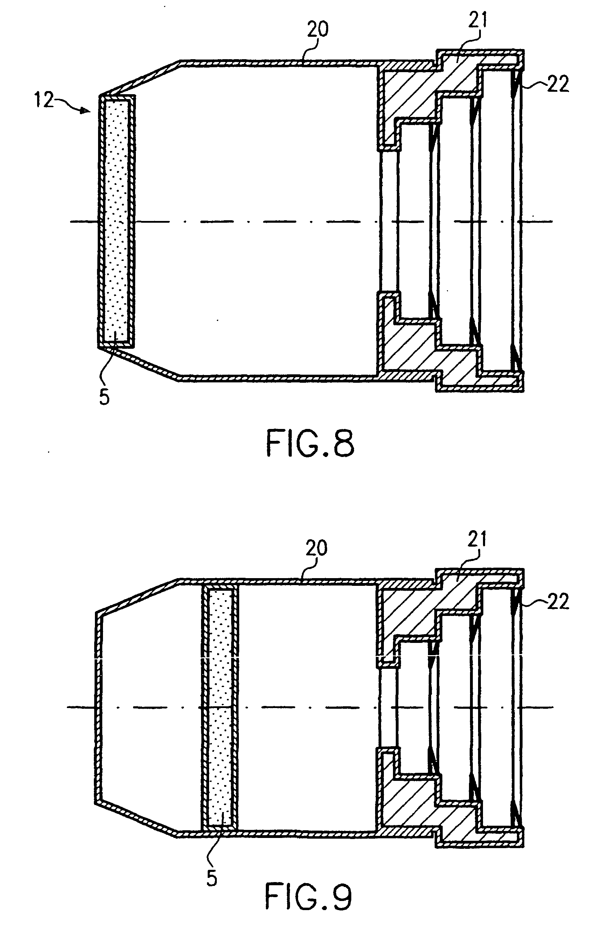

Die Fig. 8 und 9 veranschaulichen zwei abgewandelte Ausführungsformen, bei welchen der für die Erfindung wesentliche ionisierende Körper 5 aus kristallinem Salz oder dieses Salz enthaltend in einem separaten Aufsatz oder Vorsatz 20 ebenfalls auswechselbar gehalten ist. Bei der Ausführungsvariante nach Fig. 8 ist die Halterung des Körpers 5 direkt in der Luftaustrittsöffnung vorgesehen, während bei der Ausführungsform nach Fig. 9 das ionisierende Element im Innern des Aufsatzes 20 angeordnet ist. Im Bedarfsfall ermöglicht ein innenseitig abgestufter Adapterring 21 eine Umfangsanpassung an unterschiedliche Querschnitte des Luftaustrittsstutzens bei unterschiedlichen Typen von Haarföhns. Die unterschiedlichen innenseitigen Durchmesserabstufungen des Adapterrings 21 können mit Dichtlippen 22 versehen sein, so dass eine weitgehend abdichtende feste Halterung des Aufsatzes 20 am Warmlufterzeugungsgerät gewährleistet ist.

Um eine Behandlung mit dem erfindungsgemäßen Therapiegerät besonders effektiv zu gestalten, sollten alle im Folgenden kurz angegebenen Anweisung befolgt werden.

Natürlich ist es am besten, die Anwendung am ersten Tag einer Erkältung zu beginnen. Dadurch kann schon am Ende dieses Tages eine Besserung erfahren werden. Sollte eine Erkältung schon mehrere Tage bestehen, kann sie noch immer beseitigt werden, allerdings kann es bis zum Eintritt einer deutlichen Besserung bis zum nächsten Morgen dauern.

Sollte die Nase so verstopft sein, dass nicht durch sie geatmet werden kann, wird oft schon die erste Anwendung die Nase weit genug öffnen, um das Problem zu lösen. Falls dies nicht erreicht wird, kann ein übliches Nasenspray verwendet werden um die Nase zu öffnen. Die Nasenatmung ist während der Behandlung unbedingt notwendig. Dazu wird das erfindungsgemäße Therapiegerät mit der Warmluft-Ausströmseite auf das Gesicht gerichtet, so dass möglichst nur erwärmte Luft eingeatmet werden kann. Die Temperatureinstellung und der Abstand zum Gesicht sollten dazu führen, dass die Hitze durchaus als unangenehm, aber noch tolerierbar empfunden wird. Zweckmäßig ist es, ein feuchtes Tuch bereitzuhalten, um zu starke Hauterwärmung oder gar Verbrennungen zu vermeiden. Zweckmäßig kann es sein, die Lippen und das äußere der Nase ausschließlich während der Ausatmung anzufeuchten, um etwa Abkühlung zu erreichen. Die Einatmung der erwärmten Luft darf nicht unterbrochen werden, wobei das Gerät gegebenenfalls auch etwas weiter entfernt gehalten werden kann, um die Temperatureinstellung zu verändern. Wird festgestellt, dass das Gerät untolerierbar heiß eingestellt wurde, ist es am besten die Behandlung nochmals von vorne zu beginnen. Dabei wird ausschließlich heiße Luft ca. 3 Minuten lang eingeatmet und danach sollten ca. 1,5 Tassen warmes Wasser getrunken werden, wobei "warm" bedeutet, dass das Wasser mit normalen Schlucken ohne Schwierigkeiten getrunken werden kann, insbesondere in Abständen von 2 bis 3 Sekunden. Dieses zu trinkende Wasser sollte in keinem Fall durch Mikrowellenenergie aufgewärmt worden sein, da bei dieser Behandlung die natürliche Struktur der Wassermoleküle gestört wird, was zur Entstehung von freien Radikalen und radiolytischen und radiomimetischen Giftstoffen führen kann. Dieser Hinweis berücksichtigt insbesondere Erkenntnisse aus Studien, die zeigen, dass mikrowellenerhitztes Wasser zu einer Immunreaktion führen kann, die durchaus mit jener vergleichbar ist, die in der Folge einer Infektionskrankheit entsteht. Eine solche Reaktion kann bis zu 8 Stunden andauern, so dass während einer bereits bestehenden Infektion, wie einer Erkältung, das Immunsystem zusätzlicher Belastung ausgesetzt wäre, was für die Therapie der Erkältungskrankheit natürlich kontraproduktiv wäre.

Der ganz Prozess des Einatmens von ausschließlich heißer und durch das erfindungsgemäße Therapiegerät ionisierter Luft für 3 Minuten und das unmittelbar anschließende Trinken von 1,5 Tassen heißen Wassers sollte fünfmal wiederholt werden. Die Intervalle zwischen diesen Behandlungen sollten mindestens eine, jedoch nicht mehr als 1,5 Stunden betragen. Bei Einhaltung dieser Vorgaben lässt sich als Ergebnis von Versuchen in den meisten Fällen eine vollständige Genesung erreichen. Sofern jedoch Viren vorhanden sind, die in eine kristalline Phase gelangen oder schon gelangt sind, ist nicht vollständig auszuschließen, dass diese kristallisierten Viren sogar die hohen Anwendungstemperaturen überleben. Um tatsächlich alle Viren zu eliminieren sind - wie erwähnt - in der Regel fünf Anwendungen mit dem Therapiegerät nötig, und zwar auch dann, wenn nach beispielsweise 3 oder 4 Behandlungen die Symptome bereits deutlich zurückgehen. Erst nach fünf Anwendungen kann davon ausgegangen werden, dass auch die inkubierten und kristallisierten Viren in den allermeisten Fällen eliminiert wurden, so dass für die Viren keine Möglichkeit mehr besteht, sich zu erholen.

Sollte eine vorschriftsmäßige Behandlung mit dem erfindungsgemäßen Therapiegerät nicht innerhalb eines Tages zu einem vollständigen Verschwinden der Erkältungssymptome geführt haben, so ist davon auszugehen, dass bei der befallenen Person etwas anderes als (nur) eine Erkältung vorliegt. Da das Atmungssystem in seinen Reaktionen auf Erkrankungen eingeschränkt ist, können einige andere Erkrankungen ähnliche Symptome hervorrufen wie eine Erkältung. Falls diese Symptome anhalten, sollte ein Arzt aufgesucht werden.

### Literaturliste

- Lit. [1]: Anderson I, Jensen PL, Reed SE, Craig JW, Proctor DF, Adams GK: Induced Rhinovirus infection under controlled exposure to sulphur dioxide. Arch Environ Health 1977, 32: 120 - 126.
- Lit. [2]: Gregg I. Introduction: Viral Infection of the respiratory tract.
Eur L Respir Dis 1983, 64: (suppl 128).
- Lit. [3]: MacGregor S, Mayor HD: Internal Components Released from Rhinovirus and Poliovirus By Heat. J Gen Virol 1971, 10: 203 - 207.
- Lit. [4]: Larson HE, Reed SE, Tyrrell DAJ: Isolation of rhinoviruses and coronaviruses from 38 colds in adults. J Med Virol 1980, %: 221 - 229.

## Patentansprüche

1. Therapiegerät zur Behandlung von Erkältungen mit einer Einrichtung (2, 4) zur Erzeugung eines Luftstroms zwischen einer Luft-Ansaugseite und einer Luft-Ausstoßseite, wobei der Luftstrom innerhalb eines Gerätegehäuses (1) mit kristallisiertem Salz in Berührung gelangt,
**dadurch gekennzeichnet, dass**
- im Gerätegehäuse (1) eine Heizeinrichtung (3) eingebaut ist, die den Luftstrom zwischen der Luft-Ansaug- und der -Ausstoßseite auf eine mindestens der menschlichen Körperkerntemperatur entsprechende Temperatur erwärmt, und dass
- zur negativen Ionisierung des erwärmten Luftstroms ein vom Warmluftstrom vor Austritt aus dem Gerätegehäuse (1) beaufschlagter, kristallisiertes Salz enthaltender Körper (5) im Gerätegehäuse (1) gehalten ist.

2. Therapiegerät nach Anspruch 1, **dadurch gekennzeichnet, dass** als kristallisiertes Salz solches aus natürlichen Vorkommen verwendet wird.

3. Therapiegerät nach Anspruch 2, **dadurch gekennzeichnet, dass** die Warmluftstrom-Erzeugungseinrichtung als Handgerät nach Art eines Haarföhns gestaltet ist, und dass der vom Warmluftstrom beaufschlagte Körper (5) eine in diesem Luftstrom angeordnete Matrix aus dem kristallisierten Salz ist.

4. Therapiegerät nach Anspruch 3, **dadurch gekennzeichnet, dass** die Matrix aus kristallisiertem Salz in Form einer durch ein auswechselbares, scheibenförmiges Gitter gehaltene und quer zur Warmluftströmung stehende kristalline Salzcharge in relativ loser Schüttung ist.

5. Therapiegerät nach Anspruch 3, **dadurch gekennzeichnet, dass** die Matrix aus kristallisiertem Salz als röhrenförmiger Körper gestaltet und im Gerät in Axialausrichtung zur Warmluftströmung angeordnet ist und von dieser im Betrieb des Geräts durchsetzt wird.

6. Therapiegerät nach Anspruch 5, **dadurch gekennzeichnet, dass** der röhrenförmige Körper in Form einer Laval-Düse zur Konzentration und zur Erhöhung der Austrittsgeschwindigkeit des von kristallisiertem Salz ionisierten Warmluftstroms gestaltet ist.

7. Therapiegerät nach Anspruch 4, **dadurch gekennzeichnet, dass** das Gitter in der Form einzelner vernetzter Hohlrippen als Spritzteil aus warmfestem Kunststoff gestaltet ist, wobei die Hohlrippen die kristallisierte Salzmischung enthalten und auf der Zustromseite der erwärmten Luft durch eine die Salzmischung in den Hohlrippen haltende Gaze aus warmfestem Kunststoff abgedeckt sind.

8. Therapiegerät nach einem der vorstehenden Ansprüche, **dadurch gekennzeichnet, dass** im Gerät Maßnahmen zur Reduzierung von nach außen dringenden höherfrequenten elektromagnetischen Feldern (Elektrosmog) getroffen sind.

## Claims

1. A therapy device for the treatment of colds, having a means (2, 4) for producing a current of air between an air intake end and an air expulsion end, whereby the current of air comes into contact with crystallised salt within a housing (1),
**characterised in that**,
incorporated in the housing (1) there is a heating device (3) which heats the current of air between the air intake end and the air expulsion end to a temperature corresponding to at least human body temperature, and that,
for the negative ionisation of the heated air current, a body (5) containing crystallised salt is mounted within the housing (1) and is impinged by the heated air current prior to its expulsion from the housing (1).

2. A therapy device according to claim 1, **characterised in that** the crystallised salt that is used is that which is obtained from natural deposits.

3. A therapy device according to claim 2, **characterised in that** the device producing the heated air current is in the form of a hand-held appliance similar to a hair dryer, and that the body (5) impinged by the heated air current is a matrix made up of the crystallised salt and arranged within said heated air current.

4. A therapy device according to claim 3, **characterised in that** the matrix that is made up of the crystallised salt is in the form of a. relatively loosely packed measured quantity of crystalline salt held in place by a replaceable disk-shaped grid and positioned at right angles to the direction of the heated current of air.

5. A therapy device according to claim 3, **characterised in that** the matrix made up of the crystallised salt is constructed as a tubular body arranged within the device in axial alignment to the heated current of air and is infiltrated by said heated current of air during the operation of the device.

6. A therapy device according to claim 5, **characterised in that** the tubular body is in the form of a Laval nozzle in order to concentrate and increase the exit speed of the heated air current that has been ionised by the crystallised salt.

7. A therapy device according to claim 4, **characterised in that** the grid is constructed as an injection-moulded piece made of a heatproof plastic in the form of individual cross-linked concave ribs, whereby the concave ribs contain the crystallised salt mixture and, on the inflow side of the heated air current, are covered by a gauze that is made of a heatproof plastic holding the salt mixture inside the concave ribs.

8. A therapy device according to one of the preceding claims, **characterised in that**, within the device, provision is made to reduce the higher-frequency electromagnetic fields (electrosmog) that penetrate through to the outside.

## Revendications

1. Appareil de thérapie pour le traitement de refroidissements comportant un dispositif (2,4) pour la production d'un courant d'air entre un côté d'aspiration de l'air et un côté de refoulement d'air, le courant d'air venant en contact, à l'intérieur d'un boîtier (1) de l'appareil, avec un sel cristallisé,
**caractérisé en ce que**
- dans le boîtier (1) de l'appareil est monté un dispositif de chauffage (3), qui chauffe le courant d'air entre le côté d'aspiration de l'air et le côté de refoulement de l'air à une température correspondant au minimum à la température interne du corps humain, et que
- un corps (5) contenant un sel cristallisé chargé par le courant d'air chaud avant sa sortie hors du boîtier (1) de l'appareil est retenu pour l'ionisation négative du courant d'air chaud, dans le boîtier (1) de l'appareil.

2. Appareil de thérapie selon la revendication 1, **caractérisé en ce qu'**on utilise comme sel cristallisé un sel d'origine naturelle.

3. Appareil de thérapie selon la revendication 2, **caractérisé en ce que** le dispositif de production du courant d'air chaud est agencé sous la forme d'un appareil manuel réalisé à la manière d'un sèche-cheveux et que le corps (5) chargé par le courant d'air chaud est une matrice disposée dans ce courant d'air et formée par du sel cristallisé.

4. Appareil de thérapie selon la revendication 3, **caractérisé en ce que** la matrice formée par un sel cristallisé se présente sous la forme d'une charge de sel cristallin, qui est retenue par. une grille interchangeable en forme de disque et est disposée transversalement par rapport à l'écoulement d'air chaud, selon un entassement relativement lâche.

5. Appareil de thérapie selon la revendication 3, **caractérisé en ce que** la matrice constituée par un sel cristallin est agencée en tant que corps de forme tubulaire et est disposée dans l'appareil, avec une orientation axiale par rapport au courant d'air chaud, et est traversée par ce dernier lors du fonctionnement de l'appareil.

6. Appareil de thérapie selon la revendication 5, **caractérisé en ce que** le corps de forme tubulaire est agencé sous la forme d'une buse de Laval pour la concentration et pour l'accroissement de la vitesse de sortie du courant d'air chaud ionisé par le sel cristallisé.

7. Appareil de thérapie selon la revendication 4, **caractérisé en ce que** la grille est agencée sous la forme de nervures creuses individuelles maillées en tant que partie formée par injection en une matière plastique résistante à la chaleur, les nervures creuses contenant le mélange de sel cristallisé et étant recouvertes, sur le côté d'arrivée de l'air chaud, par une gaze retenant le mélange de sel dans les nervures creuses et formée d'une matière plastique résistante à la chaleur.

8. Appareil de thérapie selon l'une des revendications précédentes, **caractérisé en ce que** dans l'appareil, des dispositions sont prises pour réduire des champs électromagnétiques à haute fréquence (brouilleur électrique) qui arrivent de l'extérieur.
